# FASCICULE DE BREVET EUROPEEN

(11) **EP 3 580 199 B1**
(45) Date de publication et mention de la délivrance du brevet: **05.04.2023**
(21) Numéro de dépôt: 18703602.5
(22) Date de dépôt: 09.02.2018
(51) Int. Cl.: C07C 231/02, C07C 231/12, C07C 233/54, C07C 235/16, C07D 309/06

(54) **PROCÉDÉ DE PRÉPARATION MONOTOPE DE COMPOSÉS ORGANO-IODÉS**
VERFAHREN ZUR EIN-TOPF-HERSTELLUNG VON ORGANO-IODIERTEN VERBINDUNGEN
METHOD FOR THE ONE-POT PRODUCTION OF ORGANO-IODINATED COMPOUNDS

(30) Priorité: 10.02.2017 FR 1751121
(43) Date de publication de la demande: 18.12.2019
(73) Titulaire: Guerbet, 93420 Villepinte (FR)
(72) Inventeur: PETTA, Myriam, 95160 Montmorency (FR); PELLINGHELLI, Stephan, 95820 Bruyeres Sur Oise (FR)
(74) Mandataire: Lavoix
(86) Numéro de dépôt international: PCT/EP2018/053361
(87) Numéro de publication internationale: WO 2018/146285

(56) Documents cités:
- WO-A1-2012/175903
- GB-A- 2 496 971
- YUJIRO HAYASHI: "Pot economy and one-pot synthesis", CHEMICAL SCIENCE, vol. 7, no. 2, 1 janvier 2016 (2016-01-01) , pages 866-880, XP055426468, United Kingdom ISSN: 2041-6520, DOI: 10.1039/C5SC02913A

## Description

La présente invention concerne un procédé de préparation de composés organo-iodés. Plus particulièrement, la présente invention concerne un procédé de préparation de composés organo-iodés utiles en tant qu'intermédiaires de préparation dans la synthèse de produits de contraste iodés.

Actuellement, la majorité des procédés de synthèse des produits de contraste iodés utilisent le dichlorure d'acide 5-amino-2,4,6-triiodoisophtalique (aussi appelé DiCOCI), de formule suivante :

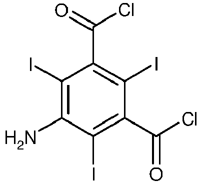

Ce composé est notamment utilisé comme produit intermédiaire dans la synthèse de nombreux produits de contraste comme l'iopamidol (Iopamiron^{®}), l'iohexol (Omnipaque^{®}), l'ioversol (Optiray^{®}), l'iomeprol (Iomeron^{®}) ou l'iobitridol (Xenetix^{®}).

Lors de la synthèse de produits de contraste iodés, il est nécessaire de réaliser de longues étapes de séparation et de purification pour aboutir à des intermédiaires de synthèse avec un bon degré de pureté. Ces étapes augmentent considérablement le temps de réalisation de la synthèse et augmentent donc ainsi le coût de mise en oeuvre des procédés de préparation des produits de contraste.

L'une des étapes de fabrication du DiCOCI est une étape de chloruration (aussi appelée chloration) de l'acide 5-amino-2,4,6-triiodoisophtalique (AATI) avec un agent chlorurant tel que le chlorure de thionyle, appelé aussi dichlorure de thionyle (SOCl₂). Cette chloruration est une étape lente (plus de 7 heures 30 de temps de réaction) et consommatrice en énergie, puisqu'elle est réalisée à une température élevée de plus de 48°C. Un excès important de l'agent chlorurant par rapport à l'AATI permet d'obtenir une cinétique plus élevée mais l'utilisation de cet excès n'est pas acceptable d'un point de vue industriel et environnemental. En effet, le chlorure de thionyle, au contact de l'eau, libère du chlorure d'hydrogène (HCl) et du dioxyde de soufre (SO₂), qui sont des gaz corrosifs et irritants. L'utilisation de catalyseurs de cette réaction de chloruration a été préconisée de façon à pouvoir réduire la quantité de SOCl₂ utilisée tout en obtenant un bon rendement industriel.

Les procédés de préparation de DiCOCI utilisés ont également comme inconvénient de générer une quantité importante d'effluents, du fait de l'utilisation de grandes quantités d'eau au moment de l'hydrolyse « précipitante » du DiCOCI obtenu par l'étape de chloruration. Par exemple, dans la demande EP 0 794 937 est décrit l'ajout de 22,2 à 33 équivalents d'eau par équivalent d'AATI (Exemples 1 à 3) ou dans la demande EP 0 773 925 est décrit l'ajout de 120 équivalents d'eau (voir exemple 1-E) pour hydrolyser le chlorure de thionyle. Enfin, ces procédés nécessitent la réalisation d'étapes de purification par précipitation, filtration et séchage, afin d'assurer une réactivité optimale et un rendement compétitif dans les étapes suivantes. Il faut aussi noter que l'étape de séchage peut s'avérer dangereuse puisque qu'elle présente un risque industriel de dégradation exothermique et doit donc être réalisée dans des conditions très maitrisées et contraignantes.

Suite à cette étape de chloruration, le DiCOCI est acylé. Cette étape est très longue puisqu'elle peut durer plusieurs dizaines d'heures (jusqu'à 70h parfois). Elle implique également des étapes de purification et d'isolement par essorage et séchage pour obtenir un intermédiaire de synthèse de produits de contraste iodés. L'étape de séchage présente le risque souligné ci-dessus. De plus, cette étape implique l'utilisation d'excès importants de certains réactifs qui, peuvent pour certains, s'avérer couteux que ce soit à leur achat ou même à leur synthèse. La manipulation de certains de ces réactifs (par exemple le DiCOCI) peut aussi poser des problèmes du fait de leur granulométrie. Pour cette seule étape d'acylation, on obtient des rendements limités à environ 87,5%. Le rendement de l'étape de chloruration est d'environ 90,5%. Le rendement de la combinaison des étapes de chloruration et d'acylation est alors d'environ 79,2%.

Il est également connu des procédés de préparation impliquant une acylation suivie d'une chloruration qui présentent les mêmes inconvénients que cités précédemment.

En particulier, la demande WO 2012/175903 décrit l'obtention d'un AATI acylé, obtenu à partir d'un large excès d'agent acylant. Ce composé intermédiaire acylé est ensuite isolé, filtré et séché avant d'être chloruré.

La demande GB 2 496 971 décrit un procédé de préparation du loforminol partant de l'AATI, ce procédé pouvant notamment comprendre une étape de formylation de l'AATI mettant en jeu un mélange d'anhydrides pour former un intermédiaire, suivie d'une étape de chloruration de cet intermédiaire. Durant ce procédé, l'intermédiaire est isolé par précipitation, filtration, rinçage à l'eau et séchage.

Par conséquent, il existe un besoin pour un procédé de préparation de produits de contraste iodés amélioré. Plus particulièrement, il existe un besoin pour un procédé de préparation de produits de contraste iodés applicable à l'échelle industrielle, plus économique, rapide et sûr.

La présente invention a pour but de fournir un procédé de préparation de produits organo-iodés, et plus particulièrement d'intermédiaires de synthèse de produits de contraste iodés, permettant de pallier aux inconvénients mentionnés ci-dessus.

La présente invention a également pour but de fournir un procédé de préparation de composés organo-iodés applicable à l'échelle industrielle, en particulier un procédé sûr, rapide, économique et acceptable d'un point de vue environnemental.

La présente invention a pour but de fournir un procédé de préparation de composés organo-iodés présentant un bon rendement, et notamment un meilleur rendement par rapport aux procédés connus.

La présente invention concerne donc un procédé de préparation d'un composé organo-iodé comprenant les étapes suivantes :
a) acylation de l'acide 2,4,6-triiodo-5-aminoisophtalique de formule (A) suivante : 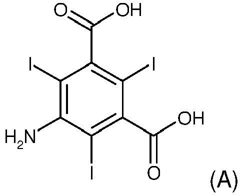 par un composé de formule générale (II) suivante :

   R₂-C(O)Cl (II)

   pour obtenir un composé intermédiaire Y ;
   puis
b) chloruration du composé intermédiaire Y obtenu à l'étape a) par un agent chlorurant pour obtenir un composé organo-iodé de formule générale (I) suivante : 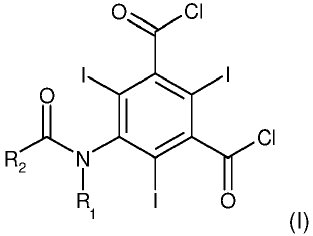 R₁ étant H, et
   R₂ étant choisi parmi le groupe constitué de :
   - (C₁-C₂₀)alkyle linéaire ou ramifié ;
   - (C₁-C₂₀)alcényle linéaire ou ramifié ;
   - (C₁-C₂₀)alcynyle linéaire ou ramifié ;
   - (C₃-C₁₀)cycloalkyle ;
   - (C₆-C₁₀)aryle ;
   - un hétérocyclyle comprenant de 3 à 10 atomes ; et
   - un hétéroaryle comprenant de 6 à 10 atomes ;
      lesdits groupes alkyle, alcényle et/ou alcynyle étant éventuellement substitués par un ou plusieurs substituant(s) choisi(s) parmi le groupe constitué des atomes d'halogène, d'oxygène et d'azote ;
      lesdits groupes alkyle, alcényle et/ou alcynyle étant éventuellement interrompus par un ou plusieurs groupe(s) choisi(s) parmi le groupe constitué de -O-, -C(O)-O- et -O-C(O)- ; et
   lesdits groupes cycloalkyle, hétérocyclyle, aryle et/ou hétéroaryle étant éventuellement substitués par un ou plusieurs substituant(s) choisi(s) parmi le groupe constitué des (C₁-C₂₀)alkyle linéaire ou ramifié, des atomes d'halogène d'oxygène et d'azote, la quantité d'agent chlorurant étant comprise entre 2 et 6 équivalents molaires par rapport à la quantité d'acide 2,4,6-triiodo-5-aminoisophtalique;
   les étapes a) et b) étant effectuées sans isolement du composé intermédiaire Y.

De façon surprenante, les inventeurs ont mis en oeuvre un procédé monotope permettant de réaliser les étapes a) d'acylation et b) de chloruration dans un même milieu réactionnel et sans nécessité d'isoler les intermédiaires réactionnels acylés obtenus à l'étape a) (composés intermédiaires Y). Ainsi, l'acylation de l'AATI est réalisée puis est directement suivie par la chloruration dudit AATI acylé aboutissant ainsi au composé organo-iodé d'intérêt avec un niveau de pureté satisfaisant et ce sans isolement de l'AATI acylé.

La succession des étapes a) d'acylation et b) de chloruration telle que selon l'invention correspond donc à un enchainement monotope.

Le procédé de préparation selon l'invention permet avantageusement d'éviter les étapes de séparation et de purification des composés intermédiaires de synthèse Y : les solvants de précipitation ou de lavage sont évités, ainsi que le traitement des eaux mères correspondantes. Le procédé de préparation selon l'invention est donc plus économique, plus rapide et plus respectueux de l'environnement.

Le procédé selon l'invention est applicable à l'échelle industrielle et permet notamment d'obtenir des rendements cumulés d'au moins 80% pour l'enchainement monotope des étapes a) et b).

Le procédé de préparation tel que selon l'invention a également comme avantage de générer des intermédiaires solubles (par « solubles », on entend que le procédé ne génère pas de cristaux).

### Définitions

Le procédé selon l'invention comprend un enchainement monotope des étapes a) et b) (également appelé « one pot » en anglais), dans lequel le composé intermédiaire Y issu de l'acylation (étape a)) n'est pas isolé avant de procéder à la chloruration (étape b)).

Un procédé de préparation ou un enchainement de réactions monotope est un procédé/enchainement dans lequel un intermédiaire de synthèse, par exemple l'AATI, subit plusieurs réactions successives et/ou simultanées dans son milieu réactionnel, en évitant les étapes de séparation et de purification des composés intermédiaires (par exemple, les composés intermédiaires Y).

Par composé « organo-iodé », on entend un composé organique comprenant au moins un atome de carbone et au moins un atome d'iode, par exemple 1, 2, 3, 4 ou 5 atomes d'iode, de préférence 3. Ledit composé organique comprend éventuellement un ou plusieurs atome(s) d'hydrogène, d'oxygène, d'azote, de soufre, de phosphore, d'halogène ou une combinaison de ces atomes. De préférence, le composé organo-iodé comprend un ou plusieurs atomes d'hydrogène (composé hydrocarboné), d'oxygène, d'azote et éventuellement de chlore.

Par « acylation », on entend une réaction chimique au cours de laquelle un groupement acyle est ajouté à un composé organique tel que l'AATI, par l'action d'un agent acylant.

Par « chloruration », également appelée chloration, on entend la substitution d'un atome et/ou d'un groupe d'atomes d'un composé organique par un atome de chlore, de préférence la substitution d'un groupement hydroxyle (-OH) par un atome de chlore (-Cl), par l'action d'un agent chlorurant ou chlorant, plus préférentiellement la double substitution des groupements hydroxyles (-OH) présents sur deux fonctions acides carboxyliques par un atome de chlore (-Cl).

Selon la présente invention, l'expression « milieu réactionnel » désigne le milieu dans lequel ont lieu les étapes a) d'acylation et b) de chloruration selon l'invention. Selon un mode de réalisation, ledit milieu réactionnel comprend au moins un solvant et au moins un réactif tel que l'AATI et/ou un agent acylant et/ou un agent chlorurant.

Par « isolement », on désigne la séparation d'un composé organo-iodé, de préférence du composé intermédiaire Y du milieu réactionnel tel que selon l'invention, éventuellement suivi de sa purification. Les méthodes de séparation et/ou de purification d'un composé organo-iodé sont connues de l'homme du métier. Peuvent par exemple être citées la filtration, la chromatographie (par exemple sur silice greffée ou non), la centrifugation, l'extraction par solvant, la cristallisation, l'adsorption (par exemple sur charbon) et la distillation.

Par "(C₁-C₂₀)alkyle", on entend un groupement hydrocarboné saturé comprenant de 1 à 20 atomes de carbone, linéaire ou ramifié. Par "ramifié", on entend qu'un ou plusieurs groupes alkyle(s) sont rattachés à un alkyle linéaire. De préférence, les alkyles sont choisis parmi le méthyle, l'éthyle, le propyle et l'isopropyle.

Par "(C₁-C₂₀)alcényle", on entend des alkyles tels que définis ci-dessus et comprenant une ou plusieurs double(s) liaison(s) carbone-carbone (insaturation éthylénique). Lorsqu'ils comprennent une seule double liaison, ils peuvent typiquement être représentés par la formule CₙH₂ₙ, n représentant le nombre d'atomes de carbone. Parmi les radicaux alcényles, on peut notamment citer les radicaux allyle ou vinyle.

Par "(C₁-C₂₀)alcynyle", on entend des alkyles tels que définis ci-dessus et comprenant une ou plusieurs triple(s) liaison(s) carbone-carbone (insaturation acétylénique). Lorsqu'ils comprennent une seule triple liaison, ils peuvent typiquement être représentés par la formule CₙH₂ₙ₋₂, n représentant le nombre d'atomes de carbone. Parmi les radicaux alcynyles, on peut notamment citer l'acétylène, les groupements éthynyle ou propynyle.

Par "(C₃-C₁₀)cycloalkyle", on entend un groupement hydrocarboné saturé et cyclique, par exemple monocyclique ou bicyclique, comprenant de 3 à 10 atomes de carbone. Parmi les cycloalkyles, on peut citer le cyclopropyle, le cyclopentyle ou le cyclohexyle.

Par "(C₆-C₁₀)aryle", on entend un groupement aromatique cyclique (monocyclique, bicyclique ou tricyclique) comprenant de 6 à 10 atomes de carbone, par exemple le phényle et le naphtyle. De préférence, le groupement aryle est un phényle.

Par "hétérocyclyle comprenant de 3 à 10 atomes", on entend un cycloalkyle tel que défini ci-dessus et dans lequel 1 ou plusieurs atomes de carbone sont remplacés par un ou plusieurs hétérotatomes tel que l'oxygène, le soufre ou l'azote. Par exemple, les hétérocyclyles comprennent 1 ou 2 atome(s) d'azote, 1 ou 2 atome(s) d'oxygène, 1 ou 2 atome(s) de soufre ou une combinaison de ceux-ci. Parmi les hétérocyclyles, on peut notamment citer l'époxyéthyle, l'oxiranyle, l'aziridinyle, le tétrahydrofuranyle, le dioxolanyle, le pyrrolidinyle, le pyrazolidinyle, l'imidazolidinyle, le tétrahydrothiophényle, le dithiolanyle, le thiazolidinyle, le tétrahydropyranyle, le dioxanyle, le morpholinyle, le pipéridyle, le pipérazinyle, le tétrahydrothiopyranyle, le dithianyle, le thiomorpholinyle, le dihydrofuranyle, le 2-imidazolinyle, le 2,-3-pyrrolinyle, le pyrazolinyle, le dihydrothiophényle, le dihydropyranyle, le pyranyle, le tétrahydropyridyle, le dihydropyridyle, le tétrahydropyrinidinyle, le dihydrothiopyranyle, et les groupes correspondants issus de la fusion avec un noyau phényle, et plus particulièrement les cycles morpholinyle, dioxalanyle, benzothiazolidinyle, pyrrolidinyle, benzopyrrolidinyle. De préférence, l'hétérocyclyle est un dioxanyle.

Par "hétéroaryle comprenant de 6 à 10 atomes", on entend un aryle tel que défini ci-dessus et dans lequel 1 ou plusieurs atomes de carbone sont remplacés par un ou plusieurs hétérotatomes tel que l'oxygène, le soufre ou l'azote. Par exemple, les hétéroaryles comprennent 1 ou 2 atome(s) d'azote, 1 ou 2 atome(s) d'oxygène, 1 ou 2 atome(s) de soufre ou une combinaison de ceux-ci. Parmi les radicaux hétéroaryles, on pourra citer le pyrazinyle, le thiényle, l'oxazolyle, le furazanyle, le pyrrolyle, le 1,2,4-thiadiazolyle, le naphthyridinyle, le pyridazinyle, le quinoxalinyle, le phtalazinyle, l'imidazo[1,2-a]pyridine, l'imidazo[2,1-b]thiazolyle, le cinnolinyle, le triazinyle, le benzofurazanyle, l'azaindolyle, le benzimidazolyle, le benzothiényle, le thiénopyridyle, le thiénopyrimidinyle, le pyrrolopyridyle, l'imidazopyridyle, le benzoazaindole, le 1,2,4-triazinyle, le benzothiazolyle, le furanyle, l'imidazolyle, l'indolyle, le triazolyle, le tétrazolyle, l'indolizinyle, l'isoxazolyle, l'isoquinolinyle, l'isothiazolyle, l'oxadiazolyle, le pyridazinyle, le pyrazolyle, le pyridyle, le pyrimidinyle, le purinyle, le quinazolinyle, le quinolinyle, l'isoquinolyle, le 1,3,4-thiadiazolyle, le thiazolyle, le carbazolyle, ainsi que les groupes correspondants issus de leur fusion ou de la fusion avec le noyau phényle.

Le terme "halogène" se réfère aux atomes du groupe 17 du tableau périodique des éléments et inclut en particulier les atomes de fluor, chlore, brome et d'iode. De préférence, l'halogène est un atome de chlore.

Selon la présente invention, l'expression "composés organo-iodés" comprend également les stéréoisomères, les hydrates, solvates, les sels organiques ou minéraux des composés organo-iodés, de préférence de formule générale (I), et des composés intermédiaires Y. Sont également comprises leurs formes tautomères, énantiomères, atropisoméres, diastéréoisomères et épimères.

### Procédé de préparation d'un composé organo-iodé

Selon un mode de réalisation, les étapes a) d'acylation et b) de chloruration du procédé selon l'invention sont réalisées sans isolement du composé intermédiaire Y dans un seul réacteur ou dans plusieurs réacteurs, préférentiellement dans un seul réacteur.

L'acylation de l'acide 2,4,6-triiodo-5-aminoisophtalique de formule (A) suivante : 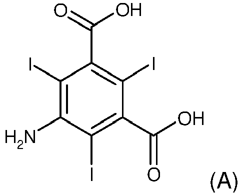 est réalisée par un chlorure d'acyle de formule générale (II).

Le procédé selon l'invention comprend les étapes suivantes :
a) acylation de l'acide 2,4,6-triiodo-5-aminoisophtalique de formule (A) suivante : 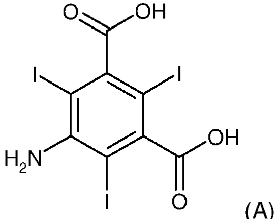 par un composé de formule générale (II) suivante :

   R₂-C(O)Cl (II)

   pour obtenir un composé intermédiaire Y ;
   puis
b) chloruration du composé intermédiaire Y par un agent chlorurant pour obtenir un composé organo-iodé de formule générale (I) suivante : 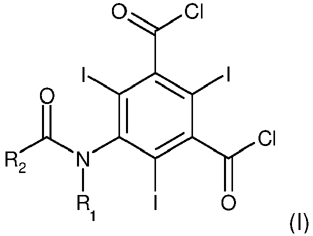
   R₁ étant H, et
   R₂ étant choisi parmi le groupe constitué de :
      - (C₁-C₂₀)alkyle linéaire ou ramifié ;
      - (C₁-C₂₀)alcényle linéaire ou ramifié ;
      - (C₁-C₂₀)alcynyle linéaire ou ramifié ;
      - (C₃-C₁₀)cycloalkyle ;
      - (C₆-C₁₀)aryle ;
      - un hétérocyclyle comprenant de 3 à 10 atomes ; et
      - un hétéroaryle comprenant de 6 à 10 atomes ;
         lesdits groupes alkyle, alcényle et/ou alcynyle étant éventuellement substitués par un ou plusieurs substituant(s) choisi(s) parmi le groupe constitué des atomes d'halogène, d'oxygène et d'azote ;
         lesdits groupes alkyle, alcényle et/ou alcynyle étant éventuellement interrompus par un ou plusieurs groupe(s) choisi(s) parmi le groupe constitué de -O-, -C(O)-O- et -O-C(O)- ; et
         lesdits groupes cycloalkyle, hétérocyclyle, aryle et/ou hétéroaryle étant éventuellement substitués par un ou plusieurs substituant(s) choisi(s) parmi le groupe constitué des (C₁-C₂₀)alkyle linéaire ou ramifié, des atomes d'halogène d'oxygène et d'azote, la quantité d'agent chlorurant étant comprise entre 2 et 6 équivalents molaires par rapport à la quantité d'acide 2,4,6-triiodo-5-aminoisophtalique, les étapes a) et b) étant effectuées sans isolement du composé intermédiaire Y.

Comme indiqué ci-dessus, lorsque R₂ est un groupe alkyle, alcényle ou alcynyle, celui-ci peut être substitué par au moins un atome d'azote, par exemple les groupes alkyles, alcényles ou alcynyles peuvent être substitués par au moins une fonction amine (primaire, secondaire ou tertiaire), ou être interrompus par un groupe -NH- ou -N((C₁-C₆)alkyle), ou encore, lorsque R₂ est un groupe alcynyle, être interrompu par au moins un atome d'azote.

Selon un mode de réalisation, R₂ est choisi parmi le groupe constitué de :
- (C₁-C₁₀)alkyle linéaire ou ramifié ;
- (C₁-C₁₀)alcényle linéaire ou ramifié ;
- (C₁-C₁₀)alcynyle linéaire ou ramifié ;
- (C₃-C₁₀)cycloalkyle ;
- un hétérocyclyle comprenant de 3 à 10 atomes ; et
   lesdits groupes alkyle, alcényle et/ou alcynyle étant éventuellement substitués par un ou plusieurs substituant(s) choisi(s) parmi le groupe constitué des atomes d'halogène;
   lesdits groupes alkyle, alcényle et/ou alcynyle étant éventuellement interrompus par un ou plusieurs groupe(s) choisi(s) parmi le groupe constitué de -O-, -C(O)-O- et -O-C(O)- ; et
   lesdits groupes cycloalkyle et/ou hétérocyclyle étant éventuellement substitués par un ou plusieurs substituant(s) choisi(s) parmi le groupe constitué des (C₁-C₁₀)alkyle linéaire ou ramifié et des atomes d'halogène.

De préférence, R₂ est choisi parmi le groupe constitué de :
- (C₁-C₂₀)alkyle linéaire ou ramifié ; et
- un hétérocyclyle comprenant de 3 à 10 atomes ;
   ledit groupe alkyle étant éventuellement substitué par un ou plusieurs substituant(s) choisi(s) parmi le groupe constitué des atomes d'halogène ;
   ledit groupe alkyle étant éventuellement interrompu par un ou plusieurs groupe(s) choisi(s) parmi le groupe constitué de -O-, -C(O)-O ou -O-C(O)- ; et ledit groupe hétérocyclyle étant éventuellement substitué par un ou plusieurs substituant(s) choisi(s) parmi le groupe constitué des (C₁-C₂₀)alkyle linéaire ou ramifié et des atomes d'halogène.
   Plus particulièrement, R₂ est choisi parmi le groupe constitué de : 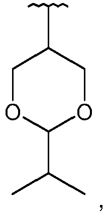 CH₃, CH₂Cl,

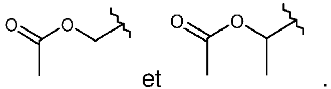

Lorsqu'il est évoqué le groupement 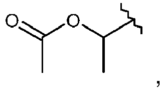 il est aussi évoqué aussi bien son isomère (R) que son isomère (S) ou encore un mélange racémique des formes isomériques (R) et (S) de ce groupement.

Selon un mode de réalisation particulier, R₂ est choisi parmi le groupe constitué de : 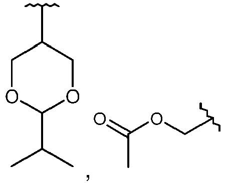 et CH₂Cl ; de préférence R₂ est :

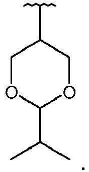

En particulier, les composés organo-iodés obtenus, préférentiellement les composés organo-iodés de formule générale (I), sont les suivants et sont utiles pour la préparation des produits de contraste correspondants, indiqués dans le tableau 1 ci-dessous.

**Tableau 1 : Composés organo-iodés de formule générale (I) et produits de contraste correspondants**

| **Formule chimique du composé organo-iodé de formule générale (I)** | **Nom du composé organo-iodé de formule générale (I)** | **Nom du produit de contraste dont il est l'intermédiaire de synthèse** |
|---|---|---|
| 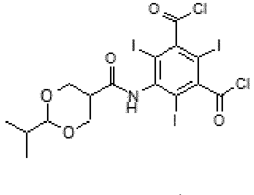 | Dichlorure de 5-[[[2-(1-méthyléthyl)-1,3-dioxan-5yl]carbonyl]amino]-2,4,6-triiodo-1,3-benzènedicarbonyle (ou DICOA) | lobitridol |
| 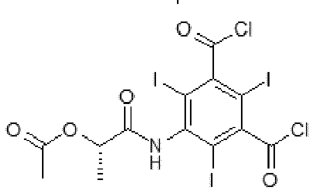 | Dichlorure de 5-{[(2S)-2-(acétyloxy)-1-oxopropyl}amino-2,4,6-triiodo-1,3-benzène dicarbonyle | lopamidol |
| 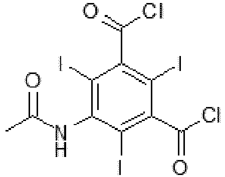 | Dichlorure de 5-(acetylamino)-2,4,6-triodobenzène-1,3-dicarbonyle | lohexol |
| | | lodixanol |
| 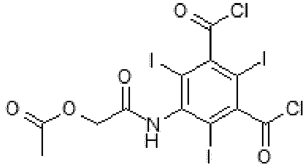 | Dichlorure de 5-{[(acétyloxy)acétyl]amino}-2,4,6-triiodobenzène-1,3-dicarbonyle | loversol |
| 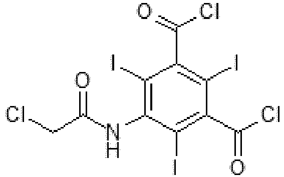 | Dichlorure de 5-[(chloroacétyl)amino]-2,4,6-triiodobenzène-1,3-dicarbonyle | loversol |
| | | |

Le composé suivant et le produit de contraste correspondant peuvent également être cités :

| **Formule chimique du composé organo-iodé** | **Nom du composé organo-iodé** | **Nom du produit de contraste dont il est l'intermédiaire de synthèse** |
|---|---|---|
| 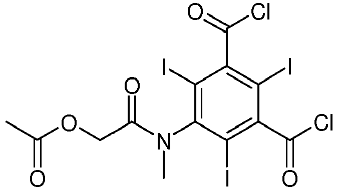 | Dichlorure de 5-{[(acétyloxy)-acétyl]méthylamino}-2,4,6-triiodobenzène-1,3-dicarbonyle | lomeprol |

Les formules chimiques de ces produits de contraste iodés sont les suivantes :

| **Nom du produit de contraste (principe actif et nom commercial associé)** | **Formule chimique** |
|---|---|
| lobitridol (XENETIX^{®}) | 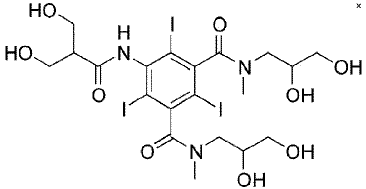 |
| lopamidol (IOPAMIRON^{®}) | 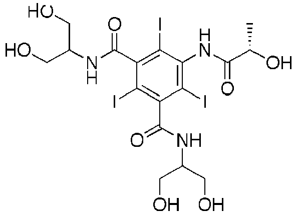 |
| lohexol (OMNIPAQUE^{®}) | 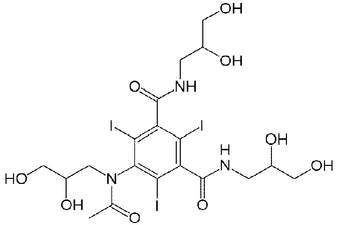 |
| lodixanol (VISIPAQUE^{®}) | 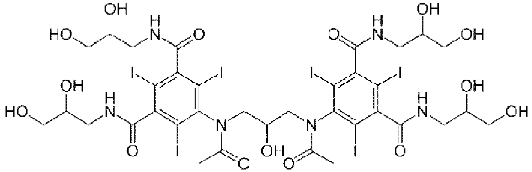 |
| loversol (OPTIJECT^{®}) | 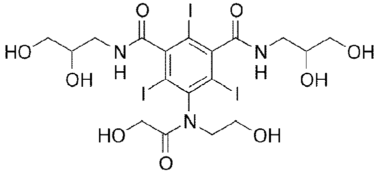 |
| lomeprol (IOMERON^{®}) | 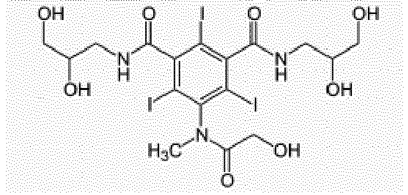 |

Selon un mode de réalisation, les composés organo-iodés obtenus, de préférence de formule générale (I), sont utiles en tant qu'intermédiaires de synthèse des produits de contraste suivants : lobitridol, lopamidol, lohexol, lodixanol, lomeprol et loversol.

Selon un mode de réalisation, les étapes a) et b) sont réalisées en présence d'un solvant aprotique et polaire.

Selon un mode de réalisation, les étapes a) et b) sont réalisées en présence d'au moins un solvant choisi parmi le groupe constitué du diméthylacétamide, du carbonate de propylène, de l'acétonitrile et du tétrahydrofurane ou d'un mélange de ceux-ci. De préférence, le solvant comprend un mélange de diméthylacétamide et de carbonate de propylène.

Les étapes a) et b) étant réalisées de façon monotope, l'étape b) est réalisée dans le milieu réactionnel résultant de l'étape a) : le(s) solvant(s) utilisé(s) ainsi que leur(s) quantité(s) sont donc préférentiellement identique(s). Selon un mode de réalisation, lors de l'étape b), un ou plusieurs solvant(s) peu(ven)t être ajouté(s) à celui(ceux) utilisé(s) pour l'étape a).

Selon un autre mode de réalisation, le ratio, en litre par kilogramme, entre la quantité de solvant (en litre) et la quantité d'acide 2,4,6-triiodo-5-aminoisophtalique (en kg) est compris entre 5 pour 1 et 1 pour 1, de préférence de 3 pour 1 ou 2,5 pour 1.

### Etape a) d'acylation

Selon un mode de réalisation, l'acylation de l'étape a) est effectuée en présence d'un agent acylant choisi parmi le DHP-COCI, le chlorure d'acétyle, le chlorure de 2-acetoxypropionyle, le chlorure de chloroacétyle, et le chlorure d'acétoxyacétyle.

Ces agents acylants ont les formules chimiques suivantes :

| **Nom de l'aqent acylant** | **Numéro CAS** | **Formule chimique** |
|---|---|---|
| Chlorure d'acide 2-(1 méthyléthyl)-1,3-dioxane-5-carboxylique (DHPCOCI) - | 116193-72-7 | 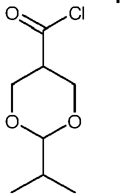 |
| Chlorure d'éthanoyle ou chlorure d'acétyle | 75-36-5 | 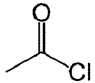 |
| Chlorure de 2-acetoxypropionyle (ou (2*S*)-1-chloro-1-oxopropan 2-yl acetate) | 13831-31-7 | 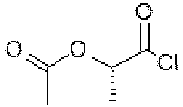 |
| Chlorure de chloroacétyle | 79-04-9 | 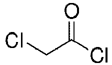 |
| Chlorure d'acétoxyacétyle | 13831-31-7 | 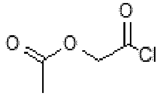 |

Selon un mode de réalisation, l'acylation de l'étape a) est effectuée en présence d'un agent acylant, de préférence un chlorure d'acyle, présent en une quantité comprise entre 1 et 1,5 équivalents molaires par rapport à la quantité d'acide 2,4,6-triiodo-5-aminoisophtalique ; préférentiellement entre 1,1 et 1,3, par exemple 1,1 ou 1,3 équivalent molaire par rapport à la quantité d'acide 2,4,6-triiodo-5-aminoisophtalique.

### Etape b) de chloruration

Selon un mode de réalisation, la chloruration de l'étape b) est effectuée en présence d'un agent chlorurant choisi parmi le groupe constitué du chlorure de thionyle, de l'oxychlorure de phosphore, du trichlorure de phosphore, du chlorure d'oxalyle, du pentachlorure de phosphore et du dichlorure de méthanoyle. Selon un mode de réalisation, la chloruration de l'étape b) est effectuée en présence d'un réactif choisi parmi le groupe constitué du chlorure de thionyle, du trichlorure de phosphore et du pentachlorure de phosphore. De préférence, l'agent chlorurant est le chlorure de thionyle.

Selon l'invention, la quantité d'agent chlorurant est comprise entre 2 et 6 équivalents molaires par rapport à la quantité d'acide 2,4,6-triiodo-5-aminoisophtalique, préférentiellement comprise entre 2,5 et 5 équivalents, plus préférentiellement entre 3 et 5 équivalents, par exemple 3,5 ou 5 équivalents, encore plus préférentiellement entre 3,2 et 4 équivalents, par rapport à la quantité d'acide 2,4,6-triiodo-5-aminoisophtalique.

### Préparation de /agent acylant

Selon un mode de réalisation, une étape de préparation de l'agent acylant est effectuée avant l'étape a), de préférence sans isolement de l'agent acylant obtenu. Selon un mode de réalisation, le procédé comprend un enchainement monotope des étapes suivantes :
- préparation de l'agent acylant ;
- étape a) d'acylation telle que définie précédemment ; et
- étape b) de chloruration telle que définie précédemment.

En particulier, la synthèse de l'agent acylant est réalisée dans le même réacteur que celui dans lequel seront réalisées les étapes a) et b) telles que selon l'invention. De manière préférentielle, l'agent acylant est préparé par chloruration de l'acide carboxylique correspondant à celui-ci. Préférentiellement, l'acide carboxylique correspondant à l'agent acylant utilisé dans le procédé de préparation selon l'invention est choisi parmi l'acide 2-(1-méthyléthyl)-1,3-dioxane-5-carboxylique, l'acide acétique, l'acide acétoxypropionique, l'acide chloroacétique et l'acide acétoxyacétique.

Selon un mode de réalisation, l'étape a) est réalisée pendant une durée de 2 à 70 heures, préférentiellement de 2 à 24h ; et/ou l'étape b) est réalisée pendant une durée de 2 à 22 heures, préférentiellement de 4 à 12h.

Selon un autre mode de réalisation, l'étape a) est réalisée à une température de 10 à 70°C, de préférence de 15 à 60°C, encore plus préférentiellement de 15 à 30°C, par exemple entre 15 et 20°C. Selon un autre mode de réalisation, l'étape b) est réalisée à une température de -15 à 30°C, préférentiellement de -10 à 10°C, encore plus préférentiellement de 0 à 10°C.

### Composés intermédiaires de préparation Y

La présente demande décrit également un composé de formule générale (Y1) suivante : 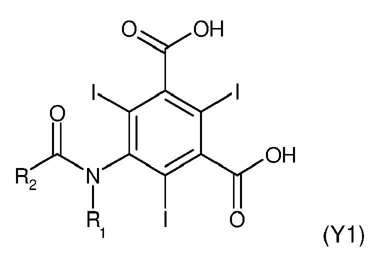
R₁ étant H, et
R₂ étant choisi parmi le groupe constitué de :
   - (C₁-C₂₀)alkyle linéaire ou ramifié ;
   - (C₁-C₂₀)alcényle linéaire ou ramifié ;
   - (C₁-C₂₀)alcynyle linéaire ou ramifié ;
   - (C₃-C₁₀)cycloalkyle ;
   - (C₆-C₁₀)aryle ;
   - un hétérocyclyle comprenant de 3 à 10 atomes ; et
   - un hétéroaryle comprenant de 6 à 10 atomes ;
      lesdits groupes alkyle, alcényle et/ou alcynyle étant éventuellement substitués par un ou plusieurs substituant(s) choisi(s) parmi le groupe constitué des atomes d'halogène, d'oxygène et d'azote;
      lesdits groupes alkyle, alcényle et/ou alcynyle étant éventuellement interrompus par un ou plusieurs groupe(s) choisi(s) parmi le groupe constitué de -O-, -C(O)-O- et -O-C(O)- ; et
      lesdits groupes cycloalkyle, hétérocyclyle, aryle et/ou hétéroaryle étant éventuellement substitués par un ou plusieurs substituant(s) choisi(s) parmi le groupe constitué des (C₁-C₂₀)alkyle linéaire ou ramifié, des atomes d'halogène d'oxygène et d'azote ;
      à condition que R₂ soit différent du groupement 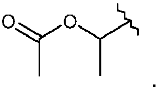
      R₂ peut être tel que défini ci-dessus pour les composés organo-iodés de formule générale (I), à condition que R₂ soit différent du groupement

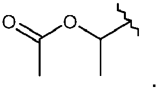

De préférence, le composé ci-dessus a l'une des formules (ay), (by), (cy) ou (dy) suivantes, la formule (ey) n'étant pas couverte par la formula générale (I):

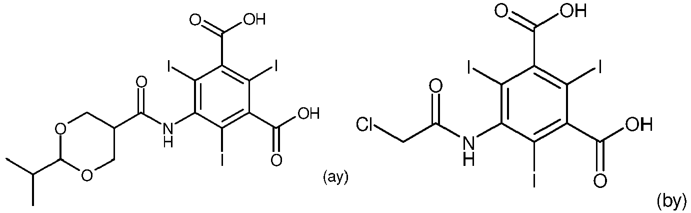

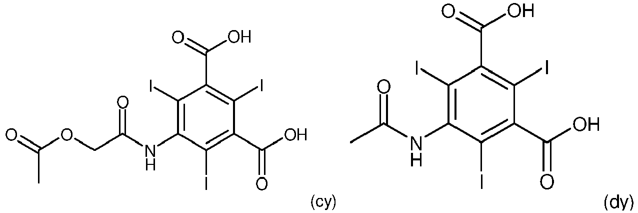

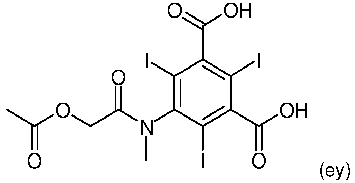

De préférence, les composés sont les composés (ay), (by) et (cy). Les composés de formule générale (Y1) correspondent notamment aux composés intermédiaires Y tels que définis ci-dessus.

La présente demande décrit également l'utilisation des composés de formule générale (Y1) telle que définie ci-dessus pour la préparation de composés organo-iodés, de préférence de formule générale (I) telle que définie ci-dessus.

La présente demande décrit également l'utilisation des composés de formule générale (Y1) telle que définie ci-dessus pour la préparation de produits de contraste, de préférence pour la préparation de produits de contraste iodés et encore plus préférentiellement pour la préparation de l'iopamidol (lopamiron^{®}), l'iohexol (Omnipaque^{®}), l'ioversol (Optiray^{®}), l'iomeprol (lomeron^{®}) ou l'iobitridol (Xenetix^{®}).

La présente invention décrit l'utilisation du composé de formule générale : 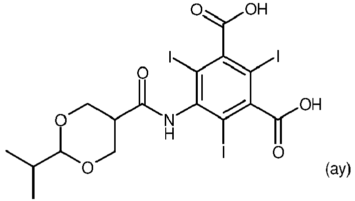
pour la préparation du iobitridol
et/ou l'utilisation du composé de formule générale 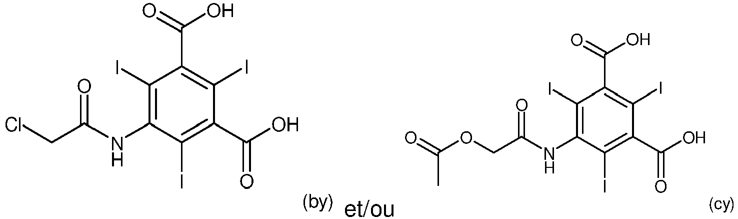
pour la préparation du ioversol et/ou l'utilisation du composé de formule générale (ey) pour la préparation du iomeprol.

Les exemples figurant ci-après sont présentés à titre illustratif et ne sont pas limitatifs de l'invention.

### EXEMPLES

### Méthodes analytiques utilisées

Les composés de synthèse sont analysés par deux techniques de Résonnance Magnétique Nucléaire : RMN du proton à 400 MHz et RMN du carbone 13 à 100 MHz. Les composés sont solubilisés dans du DMSO-d6. Les composés sont également analysés par Spectrométrie de Masse, en mode négatif et en mode positif. L'identification RMN est effectuée avec un spectromètre RMN de marque Bruker, 400Mz. L'identification MS avec un spectromètre de Masse du type Orbitrap de Marque Thermo Fischer Q-Exactive,

Par « V », on entend désigner un rapport volumique, soit le volume d'un réactif ou d'un solvant par rapport à un 1 kg d'AATI.

Par « eq. », on entend désigner un nombre d'équivalent molaire, soit le rapport entre le nombre de moles d'un réactif et le nombre de moles de l'AATI.

### Exemple 1 : Synthèse du dichlorure de 5-[[[2-(1-methylethyl)-1,3-dioxan-5yl]carbonyl]amino-2,4,6-triiodo-1,3-benzènedicarbonyl (DICOA)

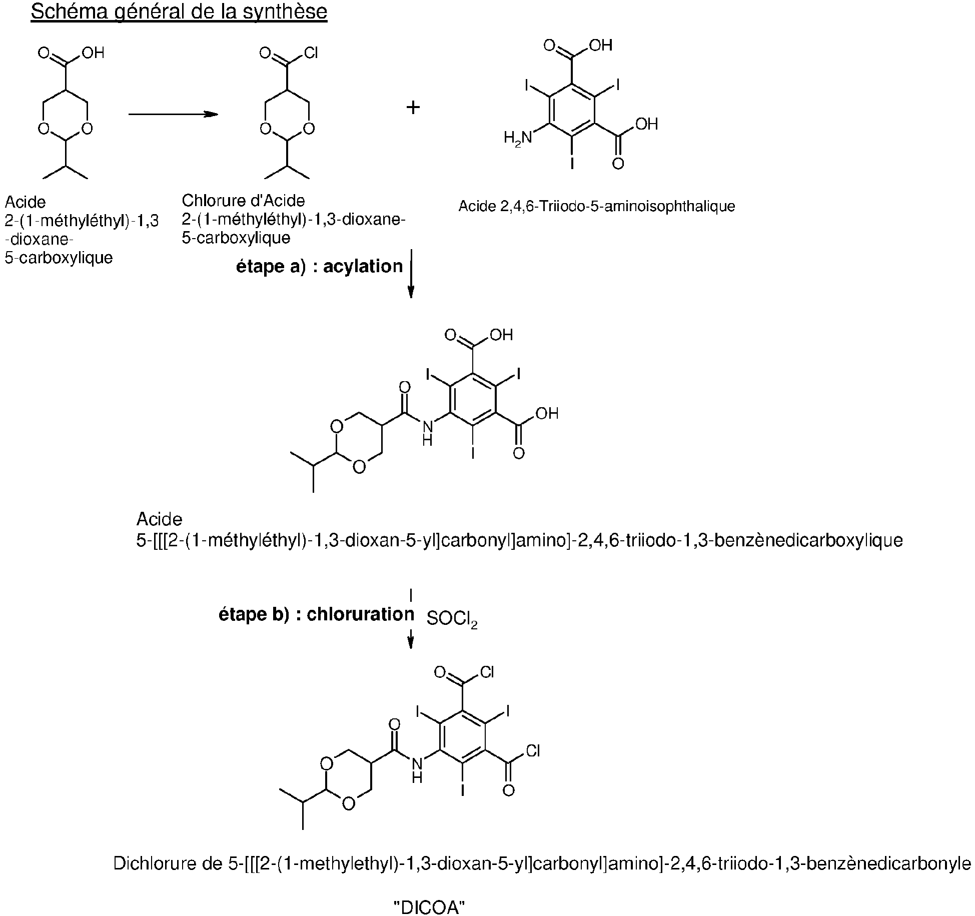

### MODE OPERATOIRE 1:

### Etape d'acylation :

Du diméthylacétamide (33,7mL ; 1,35V) et de l'acide 2-(1-méthyléthyl)-1,3-dioxane-5-carboxylique (8,7 g, 1,125 eq.) sont mélangés à 25°C jusqu'à dissolution de l'acide de départ. Le milieu réactionnel est refroidi à 0°C puis le chlorure de thionyle (6.31g, 1.06 eq./ acide de départ) est ajouté en 1h-1h30 entre 0°C et 15°C. Le milieu est agité pendant 3h à 15°C pour compléter la réaction. Du carbonate de propylène (3,7mL, 0,15V) est ajouté à 15°C dans un réacteur de 250 mL.

Ensuite, l'acide 5-amino-2,4,6-triiodoisophtalique (25 g ; 1 eq.) est introduit durant 30 minutes. A la fin de l'introduction, le milieu est réchauffé à la température de 18°C, puis la réaction d'acylation est mise en oeuvre durant 24 heures à une température comprise entre 18°C et 30°C.

L'intermédiaire acide 5-[[[2-(1-méthyléthyl)-1,3-dioxan-5-yl]carbonyl]amino]-2,4,6-triiodo-1,3-benzènedicarboxylique (composé (ay)) est alors formé.

Le milieu est ensuite fluidifié avec du carbonate de propylène (25mL : rapport volumique de 1) puis refroidi à la température de 5°C.

### Etape de chloruration :

Dès que le milieu a atteint la température de 5°C, du chlorure de thionyle (18 g ; rapport molaire équivalent de 3,5) est ajouté. L'ajout de chlorure de thionyle dure 2 heures à la température de 5°C. A la fin de l'introduction du réactif, la réaction de chloruration est mise en oeuvre durant 5 heures à température de 5°C.

Le taux de conversion alors obtenu est de 85% (s/s) en dichlorure de 5-[[[2-(1-méthyléthyl)-1,3-dioxan-5-yl]carbonyl]amino]-2,4,6-triiodo-1,3 benzènedicarbonyl.

Le milieu réactionnel est lentement ajouté sur un mélange eau/éthanol/acétate de sodium. Le solide obtenu est filtré, lavé, séché puis analysé.

Le rendement obtenu est de 82%.

### RESULTATS ANALYTIQUES :

### Résonance Magnétique Nucléaire :

**¹H NMR (400 MHz, DMSO-d6) δ :** 10.4 (s, 1H), 4.3 (d, J = 4.9 Hz, 1H), 4.3 (m, 2H), 3.9 (t, J = 11.4 Hz, 2H), 3.0 (ddt, J = 11.1, 6.9, 4.6 Hz, 1H), 1.7 (dhept, J = 6.9, 4.8 Hz, 1H), 0.9 (d, J = 6.8 Hz, 6H).

¹³C **NMR (101 MHz, DMSO-d6) δ:** 169.4-169.8, 168.3, 149.7-150.3, 143.1-143.9, 105.0, 87.1-102.0, 69.0, 41.9, 32.5, 17.3.

### Spectrométrie de masse :

**[M** - **H]⁻** = 749.7321 uma (masse exacte = 749.7310 uma)
**[M** + **H]⁺** = 751.7453 uma (masse exacte = 751.7456 uma)

### MODE OPERATOIRE 2 :

### Etape d'acylation :

Du diméthylacétamide (40mL ; 1,6V) et de l'acide 2-(1-méthyléthyl)-1,3-Dioxane-5-carboxylique (10,2g, 1,3 eq.) sont introduits dans un réacteur de 250 mL de volume. Le milieu réactionnel est refroidi à 0°C puis le chlorure de thionyle (5.4g 1.06 eq./ acide de départ) est ajouté en 1h-1h30 entre 0°C et 15°C. Le milieu est agité pendant 3h à 15°C pour compléter la réaction. Ensuite, l'acide 5-amino-2,4,6-triiodoisophtalique (25g, 1 eq.) est introduit durant 30 minutes.

A la fin de l'introduction, le milieu est réchauffé à la température de 18°C, puis la réaction d'acylation est mise en oeuvre durant 70 heures à une température de 18°C.

L'intermédiaire acide 5-[[[2-(1-méthyléthyl)-1,3-dioxan-5-yl]carbonyl]amino]-2,4,6-triiodo-1,3-benzènedicarboxylique (composé (ay)) est alors formé.

### Etape de chloruration :

Le milieu réactionnel est ensuite refroidi jusqu'à la température de -10°C. Dès que le milieu a atteint la température de -10°C, du chlorure de thionyle (26,6g, 5 eq.) est ajouté. L'ajout de chlorure de thionyle dure 2 heures, à la température de -10 °C. A la fin de l'introduction du réactif, la réaction de chloruration est mise en oeuvre durant 12 heures à la température de -10°C.

Le taux de conversion alors obtenu est de 86% (s/s) en dichlorure de 5-[[[2-(1-méthyléthyl)-1,3-dioxan-5-yl]carbonyl]amino]-2,4,6-triiodo-1,3-benzènedicarbonyle.

Le milieu réactionnel est ensuite ajouté lentement sur un mélange eau/éthanol/ acétate de sodium. Le solide obtenu est filtré, lavé, séché puis analysé.

Le rendement obtenu est de 84,5%.

### RESULTATS ANALYTIQUES :

### Résonance Magnétique Nucléaire :

**¹H NMR (400 MHz, DMSO-d6) δ :** 10.4 (s, 1H), 4.3 (d, J = 4.9 Hz, 1H), 4.3 (m, 2H), 3.9 (t, J = 11.4 Hz, 2H), 3.0 (ddt, J = 11.1, 6.9, 4.6 Hz, 1H), 1.7 (dhept, J = 6.9, 4.8 Hz, 1H), 0.9 (d, J = 6.8 Hz, 6H).

**¹³C NMR (101 MHz, DMSO-d6) δ** : 169.4-169.8, 168.3, 149.7-150.3, 143.1-143.9, 105.0, 87.1-102.0, 69.0, 41.9, 32.5, 17.3.

### Spectrométrie de masse :

[ M - H ]⁻ = 749.7321 uma (masse exacte = 749.7310 uma)
[ M + H ]⁺ = 751.7453 uma (masse exacte = 751.7456 uma)

### Exemple 2 : Synthèse du dichlorure de 5-(acetylamino)-2,4,6-triodobenzène-1,3-dicarbonyle (intermédiaire de synthèse du lohexol/lodixanol)

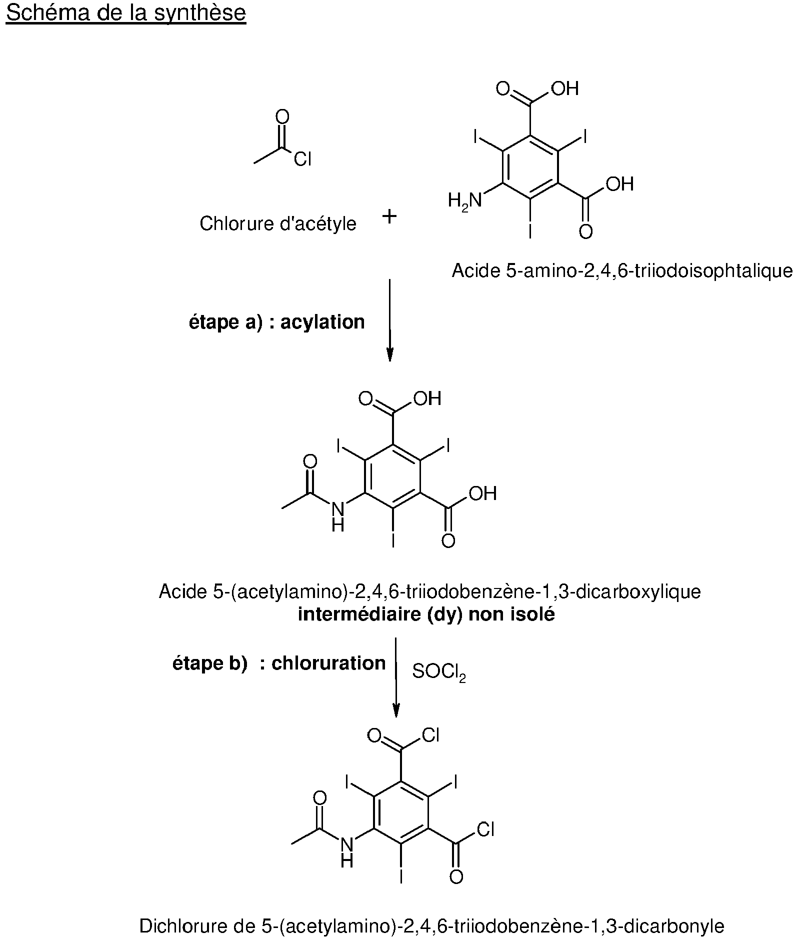

### Etape d'acylation :

Du diméthylacétamide (67,5mL ; 1,35V) et du carbonate de propylène (7,5mL ; 0,15V) sont mélangés dans un réacteur de 250 mL de volume.

Du chlorure d'acétyle (7,7g ; 1,1 eq.) est ensuite ajouté au mélange. Après refroidissement jusqu'à la température de 15°C, l'acide 5-amino-2,4,6-triiodoisophtalique (50 g ; 1 eq.) est introduit durant 30 minutes. A la fin de l'introduction, le milieu est réchauffé à température de 18°C, puis la réaction d'acylation est mise en oeuvre durant 36 heures à une température de 18°C.

L'intermédiaire acide 5-(acétylamino)-2,4,6-triiodobenzène-1,3-dicarboxylique (composé (dy)) est alors formé.

Le milieu est ensuite fluidifié avec du diméthylacétamide (50 mL ; 1V) et du carbonate de propylène (50 mL ; 1V). Puis le milieu est refroidi à une température de 5°C.

### Etape de chloruration :

Dès que le milieu a atteint la température de 5°C, du chlorure de thionyle (52,8 g ; 5 eq.) est ajouté. L'ajout de chlorure de thionyle dure 2 heures à température de 5°C. A la fin de l'introduction du réactif, la réaction de chloruration est mise en oeuvre durant 5 heures à température de 5°C.

Le taux de conversion alors obtenu est de 85% (s/s) en dichlorure de 5-(acetylamino)-2,4,6-triiodobenzène-1,3-dicarbonyle.

Le milieu réactionnel est lentement ajouté sur un mélange eau/éthanol/acétate de sodium. Le solide obtenu est filtré, lavé, séché puis analysé.

Le rendement obtenu est de 60%.

### RESULTATS ANALYTIQUES :

### Résonance Magnétique Nucléaire :

**¹H NMR (400 MHz, DMSO-d6) δ** : 10.1 (s, 1H), 2.1 (s, 3H).

**¹³C NMR (101 MHz, DMSO-d6) δ :** 168.3, 144.1-150.2, 86.8-102.2, 169.5-169.8, 144.8, 23.4.

### Spectrométrie de masse :

**[M** - **H]⁻** = 635.6634 uma (masse exacte = 635.6630 uma)
**[M** + **Na]⁺** = 659.6593 uma (masse exacte = 659.6594 uma)

### Exemple 3 : Synthèse du dichlorure de 5-{[(2S)-2-(acetyloxy)-1-oxopropyl}amino-2,4,6-triiodo-1,3-benzène dicarbonyle (intermédiaire de synthèse du lopamidol)

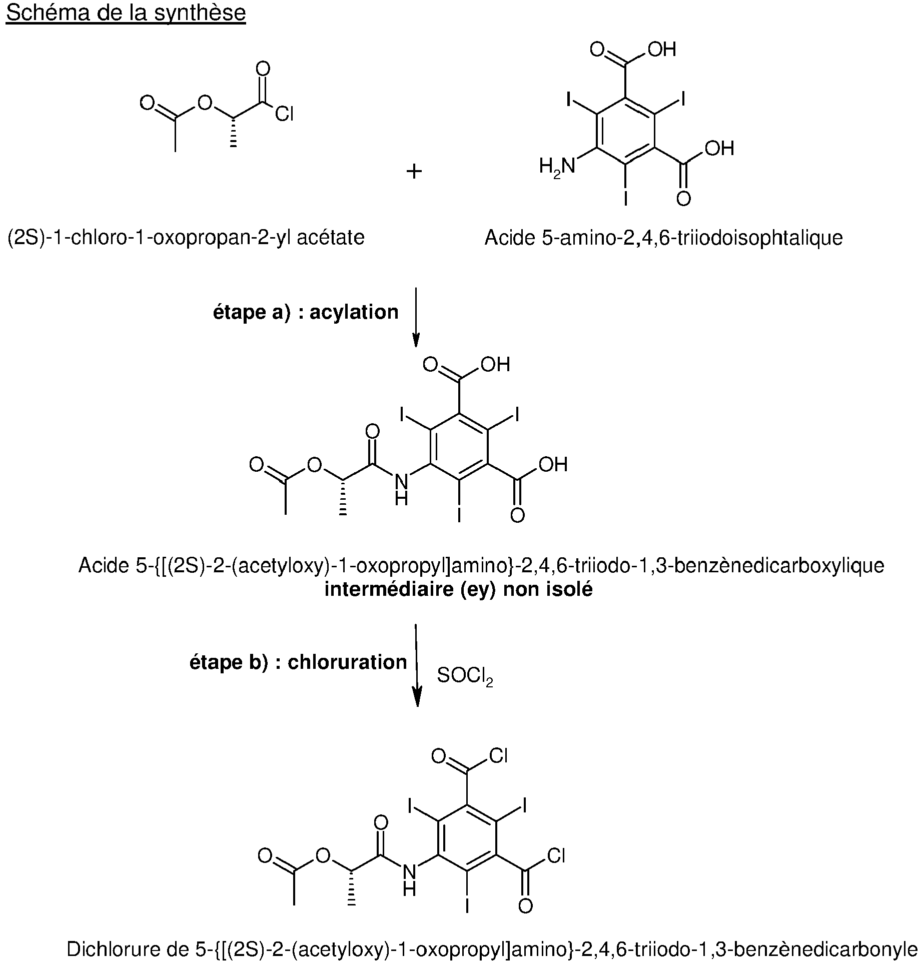

### Etape d'acylation :

Du diméthylacétamide (34 mL ; 1,35V) et du carbonate de propylène (3,75 mL ; 0,15V) sont mélangés dans un réacteur de 250 mL de volume.

Du (2S)-1-chloro-1-oxopropan-2-yl acétate (7,5 g ; 1,1 eq.) est ensuite ajouté au mélange. Après refroidissement jusqu'à la température de 15°C, l'acide 5-amino-2,4,6-triiodoisophtalique (25 grammes ; 1 eq.), est introduit durant 30 minutes. A la fin de l'introduction, le milieu est réchauffé à température de 18°C, puis la réaction d'acylation est mise en oeuvre durant 66 heures à une température de 18°C.

L'intermédiaire acide 5-{[(2S)-2-(acetyloxy)-1-oxopropyl}amino-2,4,6-triiodo-1,3-benzendicarboxylique (composé (ey)) est alors formé.

Le milieu est ensuite dilué avec du carbonate de propylène (25 mL, 1V). Puis le milieu est refroidi à une température de 5°C.

### Etape de chloruration :

Dès que le milieu a atteint la température de 5°C, du chlorure de thionyle (18,5 g ; 3,5 eq.) est ajouté. L'ajout de chlorure de thionyle dure 1 heure à température de 5°C. A la fin de l'introduction du réactif, la réaction de chloruration est mise en oeuvre durant 5 heures à température de 5°C.

Le taux de conversion alors obtenu est de 87% (s/s) en dichlorure de 5-{[(2S)-2-(acetyloxy)-1-oxopropyl}amino-2,4,6-triiodo-1,3-benzendicarbonyle.

Le milieu réactionnel est lentement ajouté sur un mélange eau/éthanol/acétate de sodium. Le solide obtenu est filtré, lavé, séché puis analysé.

Le rendement obtenu est de 70%.

### RESULTATS ANALYTIQUES :

### Résonance Magnétique Nucléaire :

**¹H NMR (400 MHz, DMSO-d6) δ** : 10.3 (s, 1H), 5.2 (q, J = 6.9 Hz, 1H), 2.1 (s, 3H), 1.5 (d, J = 6.9Hz, 3H).

**¹³C NMR (101 MHz, DMSO-d6) δ** : 142.9-150.4, 87.2-101.9, 168.5-170.0, 169.9, 168.9, 69.9, 21.3, 18.0.

### Spectrométrie de masse :

**[M** - **H]⁻ =** 707.6847 uma (masse exacte = 707.6841 uma)
**[M** + **Na]⁺ =** 731.6806 uma (masse exacte = 731.6806 uma)

### Exemple 4 : Synthèse du dichlorure de 5-{[(acetyloxy)acetyl]amino}-2,4,6-triiodobenzèn-1,3-dicarbonyle (intermédiaire de synthèse du loversol)

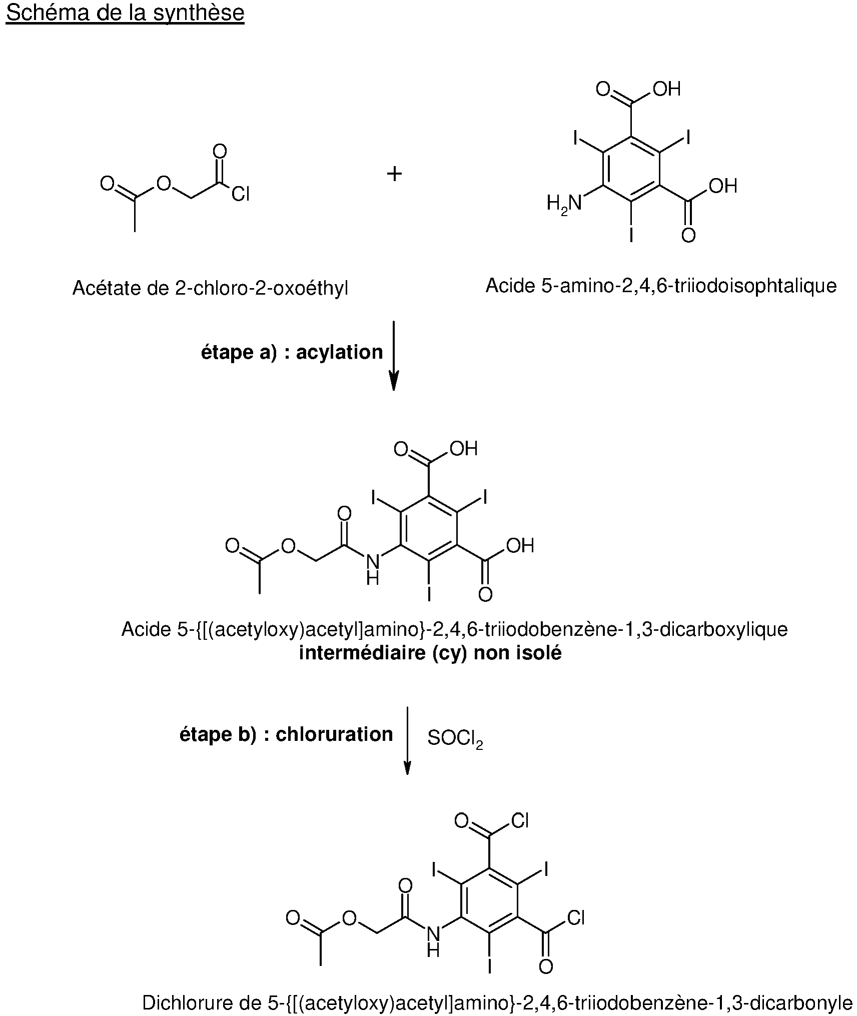

### Etape d'acylation :

Du diméthylacétamide (67,5 mL, 1,35V) et du carbonate de propylène (7,5 mL ; 0,15V) sont mélangés dans un réacteur de 250 mL de volume.

Du 2-chloro-2-oxoéthyl acétate (13,3 g ; 1,1 eq.) est ensuite ajouté au mélange. Après refroidissement jusqu'à la température de 15°C, l'acide 5-amino-2,4,6-triiodoisophtalique (50 g ; 1 eq.) est introduit durant 45 minutes. A la fin de l'introduction le milieu est réchauffé à température de 18°C, puis la réaction d'acylation est mise en oeuvre durant 65 heures à une température de 18°C.

L'intermédiaire acide de 5-{[(acetyloxy)acétyl]amino}-2,4,6-triiodobenzène-1,3-dicarboxylique (composé (cy)) est alors formé.

Le milieu est ensuite dilué avec du carbonate de propylène (50 mL ; 1V). Puis le milieu est refroidi à une température de 5°C.

### Etape de chloruration :

Dès que le milieu a atteint la température de 5°C, du chlorure de thionyle (37 g ; 3,5 eq.) est ajouté. L'ajout de chlorure de thionyle dure 2 heures à température de 5°C. A la fin de l'introduction du réactif, la réaction de chloruration est mise en oeuvre durant 4 heures à température de 5°C.

Le taux de conversion alors obtenu est de 90,5 % (s/s) en dichlorure de 5-{[(acétyloxy)acétyl]amino}-2,4,6-triiodobenzène-1,3-dicarbonyle.

Le milieu réactionnel est lentement ajouté sur un mélange eau/éthanol/acétate de sodium. Le solide obtenu est filtré, lavé, séché puis analysé. Le rendement obtenu est de 87%.

### RESULTATS ANALYTIQUES :

### Résonance Magnétique Nucléaire :

**¹H NMR (400 MHz, DMSO-d6) δ :** 10.4 (s, 1H), 4.7 (s, 2H), 2.2 (s, 3H).

**¹³C NMR (101 MHz, DMSO-d6) δ** : **143**.0-150.3, 87.3-102.0, 169.4-169.8, 170.1, 166.1, 62.6, 21.0.

### Spectrométrie de masse :

**[M - H]⁻ =** 693.6691 uma (masse exacte = 693.6684 uma)
**[M + Na]⁺ =** 717.6645 uma (masse exacte = 717.6649 uma)

### Exemple 5 : Synthèse du dichlorure de 5-[(chloroacétyl)amino]-2,4,6-triiodobenzène-1,3-dicarbonyle (intermédiaire de synthèse du loversol)

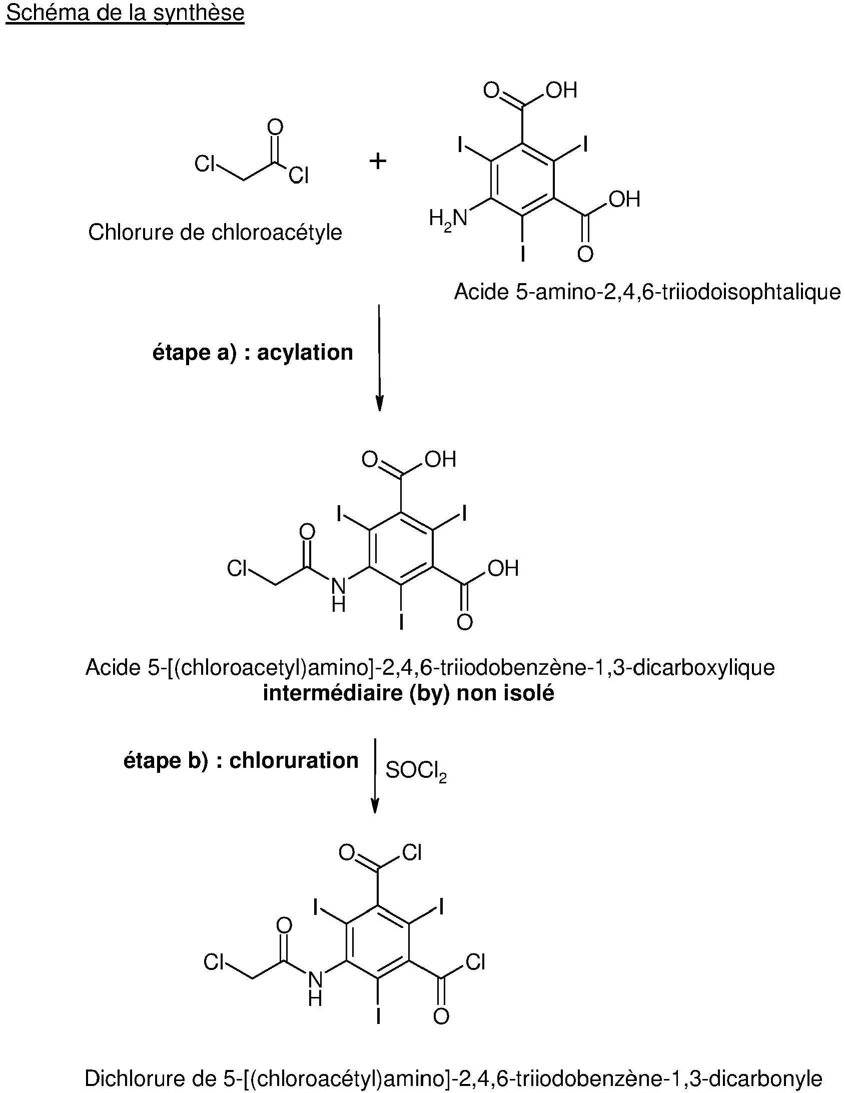

### Etape d'acylation :

Du diméthylacétamide (67,5 mL ; 1,35V) et du carbonate de propylène (7,5 mL ; 0,15V) sont mélangés dans un réacteur de 250 mL de volume.

Du chlorure de chloroacétyle (11 g ; 1,1 eq.) est ensuite ajouté au mélange. Après refroidissement jusqu'à la température de 15°C, l'acide 5-amino-2,4,6-triiodoisophtalique (50 g ; 1 eq.), est introduit par portions, soit environ 3,4 g toutes les deux minutes, durant 30 minutes. A la fin de l'introduction le milieu est réchauffé à température de 18°C, puis la réaction d'acylation est mise en oeuvre durant 40 heures à une température de 18°C.

L'intermédiaire acide 5-[(chloroacetyl)amino]-2,4,6-triiodobenzène-1,3-dicarboxylique (composé (by)) est alors formé.

Le milieu est ensuite fluidifié avec du diméthylacétamide (50 mL ; 1V) et du carbonate de propylène (50 mL ; 1V). Puis le milieu est refroidi à une température de 5°C.

### Etape de chloruration :

Dès que le milieu a atteint la température de 5°C, du chlorure de thionyle (52,9 g ; 5 eq.) est ajouté. L'ajout de chlorure de thionyle dure 3 heures à température de 5°C. A la fin de l'introduction du réactif, la réaction de chloruration est mise en oeuvre durant 18 heures à température de 5°C.

Le taux de conversion alors obtenu est de 88 % (s/s) en dichlorure de 5-[(chloroacetyl)amino]-2,4,6-triiodobenzène-1,3-dicarbonyle.

Le milieu réactionnel est lentement ajouté sur un mélange eau/éthanol/acétate de sodium. Le solide obtenu est filtré, lavé, séché puis analysé. Le rendement obtenu est de 86%.

### Résultats analytiques :

### Résonance Magnétique Nucléaire

**¹H NMR (400 MHz, DMSO-d6) δ :** 10.6 (s, 1H), 4.4 (s, 2H).

**¹³C NMR (101 MHz, DMSO-d6) δ :** 142.9-150.3, 87.5-101.8, 169.4-169.8, 164.8, 98.4, 43.3.

### Spectrométrie de masse :

**[M** - **H]⁻** = 669.6245 uma (masse exacte = 669.6240 uma)
**[M** + **Na]⁺** = 693.6202 uma (masse exacte = 693.6205 uma)

## Revendications

1. Procédé de préparation d'un composé organo-iodé comprenant les étapes suivantes :
a) acylation de l'acide 2,4,6-triiodo-5-aminoisophtalique de formule (A) suivante : 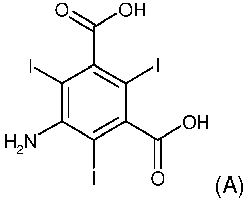 par un composé de formule générale (II) suivante :
R₂-C(O)Cl (II)
pour obtenir un composé intermédiaire Y ;
puis
b) chloruration du composé intermédiaire Y par un agent chlorurant pour obtenir un composé organo-iodé de formule générale (I) suivante : 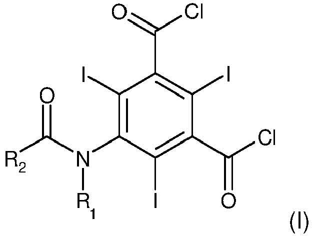
R₁ étant H, et
R₂ étant choisi parmi le groupe constitué de :
- (C₁-C₂₀)alkyle linéaire ou ramifié ;
- (C₁-C₂₀)alcényle linéaire ou ramifié ;
- (C₁-C₂₀)alcynyle linéaire ou ramifié ;
- (C₃-C₁₀)cycloalkyle ;
- (C₆-C₁₀)aryle ;
- un hétérocyclyle comprenant de 3 à 10 atomes ; et
- un hétéroaryle comprenant de 6 à 10 atomes ;
lesdits groupes alkyle, alcényle et/ou alcynyle étant éventuellement substitués par un ou plusieurs substituant(s) choisi(s) parmi le groupe constitué des atomes d'halogène, d'oxygène et d'azote ;
lesdits groupes alkyle, alcényle et/ou alcynyle étant éventuellement interrompus par un ou plusieurs groupe(s) choisi(s) parmi le groupe constitué de -O-, -C(O)-O- et -O-C(O)- ; et
lesdits groupes cycloalkyle, hétérocyclyle, aryle et/ou hétéroaryle étant éventuellement substitués par un ou plusieurs substituant(s) choisi(s) parmi le groupe constitué des (C₁-C₂₀)alkyle linéaire ou ramifié, des atomes d'halogène d'oxygène et d'azote, la quantité d'agent chlorurant étant comprise entre 2 et 6 équivalents molaires par rapport à la quantité d'acide 2,4,6-triiodo-5-aminoisophtalique,
les étapes a) et b) étant effectuées sans isolement du composé intermédiaire Y.

2. Procédé de préparation selon la revendication 1, dans lequel les étapes a) et b) sont réalisées dans un seul réacteur.

3. Procédé selon la revendication 1 ou 2, dans lequel R₂ est choisi parmi le groupe constitué de : 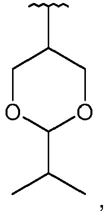 CH₃, CH₂Cl,

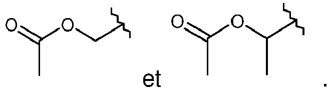

4. Procédé de préparation selon l'une quelconque des revendications précédentes, dans lequel les étapes a) et b) sont réalisées en présence d'au moins un solvant choisi parmi le groupe constitué du diméthylacétamide, du carbonate de propylène, de l'acétonitrile et du tétrahydrofurane ou d'un mélange de ceux-ci.

5. Procédé de préparation selon l'une quelconque des revendications précédentes, dans lequel la chloruration de l'étape b) est effectuée en présence d'un agent chlorurant choisi parmi le groupe constitué du chlorure de thionyle, de l'oxychlorure de phosphore, du trichlorure de phosphore, du chlorure d'oxalyle, du pentachlorure de phosphore et du dichlorure de méthanoyle.

## Patentansprüche

1. Verfahren zur Herstellung einer Organoiodverbindung, das die folgenden Schritte umfasst:
a) Acylierung von 2,4,6-Triiod-5-aminoisophthal-säure der folgenden Formel (A): 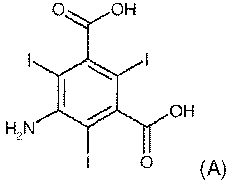 mit einer Verbindung der folgenden allgemeinen Formel (III):
R₂-C(O)Cl (II)
unter Erhalt einer Zwischenverbindung Y;
dann
b) Chlorierung der Zwischenverbindung Y mit einem Chlorierungsmittel unter Erhalt einer Organoiodverbindung der folgenden allgemeinen Formel (I): 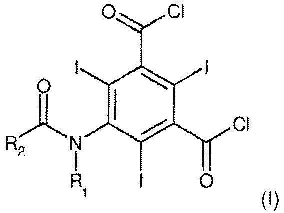 wobei
R₁ für H steht und
R₂ aus der Gruppe bestehend aus
- linearem oder verzweigtem (C₁-C₂₀)Alkyl;
- linearem oder verzweigtem (C₁-C₂₀)Alkenyl;
- linearem oder verzweigtem (C₁-C₂₀)Alkinyl;
- (C₃-C₁₀)Cycloalkyl;
- (C₆-C₁₀) Aryl;
- einem Heterocyclyl mit 3 bis 10 Atomen und
- einem Heteroaryl mit 6 bis 10 Atomen ausgewählt ist;
wobei die Alkyl-, Alkenyl- und/oder Alkinylgruppen gegebenenfalls durch einen oder mehrere Substituenten, die aus der Gruppe bestehend aus Halogen-, Sauerstoff- und Stickstoffatomen ausgewählt sind, substituiert sind;
wobei die Alkyl-, Alkenyl- und/oder Alkinylgruppen gegebenenfalls durch eine oder mehrere Gruppen, die aus der Gruppe bestehend aus -O-, -C(O)-O- und -O-C(O)- ausgewählt sind, unterbrochen sind und
die Cycloalkyl-, Heterocyclyl-, Aryl- und/oder Heteroarylgruppen gegebenenfalls durch einen oder mehrere Substituenten, die aus der Gruppe bestehend aus linearem oder verzweigtem (C₁-C₂₀)Alkyl und Halogen-, Sauerstoff- und Stickstoffatomen ausgewählt sind, substituiert sind, wobei die Menge an Chlorierungsmittel zwischen 2 und 6 Moläquivalenten, bezogen auf die Menge an 2,4,6-Triiod-5-aminoisophthalsäure, beträgt,
wobei die Schritte a) und b) ohne Isolierung der Zwischenverbindung Y durchgeführt werden.

2. Verfahren zur Herstellung nach Anspruch 1, wobei die Schritte a) und b) in einem einzigen Reaktor durchgeführt werden.

3. Verfahren nach Anspruch 1 oder 2, wobei R₂ aus der Gruppe bestehend aus 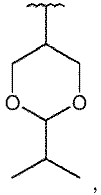 CH₃, CH₂Cl, 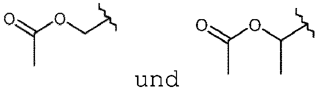 ausgewählt ist.

4. Herstellungsverfahren nach einem der vorhergehenden Ansprüche, bei dem die Schritte a) und b) in Gegenwart von mindestens einem Lösungsmittel aus der Gruppe bestehend aus Dimethylacetamid, Propylencarbonat, Acetonitril und Tetrahydrofuran oder einer Mischung davon durchgeführt werden.

5. Herstellungsverfahren nach einem der vorhergehenden Ansprüche, bei dem die Chlorierung von Schritt b) in Gegenwart eines Chlorierungsmittels aus der Gruppe bestehend aus Thionylchlorid, Phosphoroxidchlorid, Phosphortrichlorid, Oxalylchlorid, Phosphorpentachlorid und Methanoyldichlorid durchgeführt wird.

## Claims

1. Process for the preparation of an organo-iodized compound, comprising the following steps:
a) acylation of 2,4,6-triiodo-5-aminoisophthalic acid with the following formula (A): 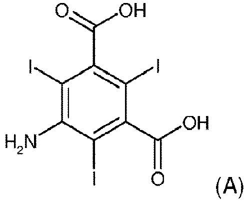 by a compound with the following general formula (II):
R₂-C(O)Cl (II)
in order to obtain an intermediate compound Y;
then
b) chlorination of the intermediate compound Y by a chlorinating agent in order to obtain an organo-iodized compound with the following general formula (I): 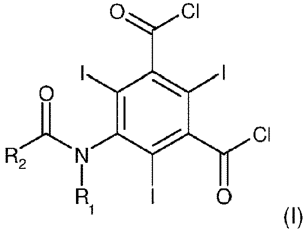
R₁ being H, and
R₂ being selected from the group constituted by:
- linear or branched (C₁-C₂₀) alkyl;
- linear or branched (C₁-C₂₀)alkenyl;
- linear or branched (C₁-C₂₀) alkynyl;
- (C₃-C₁₀)cycloalkyl;
- (C₆-C₁₀)aryl;
- a heterocyclyl comprising from 3 to 10 atoms; and
- a heteroaryl comprising from 6 to 10 atoms;
said alkyl, alkenyl and/or alkynyl groups optionally being substituted with one or more substituent (s) selected from the group constituted by atoms of halogen, oxygen and nitrogen;
said alkyl, alkenyl and/or alkynyl groups optionally being interrupted by one or more group(s) selected from the group constituted by -O-, -C(O)-O- and -O-C(O)-; and
said cycloalkyl, heterocyclyl, aryl and/or heteroaryl groups optionally being substituted with one or more substituent(s) selected from the group constituted by linear or branched (C₁-C₂₀)alkyl, and halogen, oxygen and nitrogen atoms, the amount of chlorinating agent being between 2 and 6 molar equivalents relative to the amount of 2,4,6-triiodo-5-aminoisophthalic acid,
the steps a) and b) being carried out without isolation of the intermediate compound Y.

2. Preparation process according to Claim 1, in which the steps a) and b) are carried out in a single reactor.

3. Process according to Claim 1 or 2, in which R₂ is selected from the group constituted by: 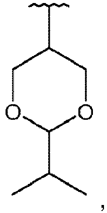 CH₃, CH₂Cl,

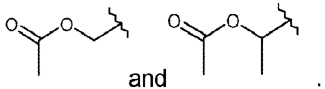

4. Preparation process according to any one of the preceding claims, in which the steps a) and b) are carried out in the presence of at least one solvent selected from the group constituted by dimethylacetamide, propylene carbonate, acetonitrile and tetrahydrofuran or a mixture thereof.

5. Preparation process according to any one of the preceding claims, in which the chlorination of step b) is carried out in the presence of a chlorinating agent selected from the group constituted by thionyl chloride, phosphorus oxychloride, phosphorus trichloride, oxalyl chloride, phosphorus pentachloride and methanoyl dichloride.
